# EUROPEAN PATENT APPLICATION

(11) **EP 2 441 392 A1**
(43) Date of publication of application: **18.04.2012**
(21) Application number: 10786119.7
(22) Date of filing: 04.06.2010
(51) Int. Cl.: A61B 8/08

(54) **ULTRASONIC DIAGNOSIS DEVICE AND INTIMA-MEDIA THICKNESS MEASURING METHOD THEREFOR**

(30) Priority: 10.06.2009 JP 2009139319
(71) Applicant: Hitachi Medical Corporation, Chiyoda-ku Tokyo 101-0021 (JP)
(72) Inventor: CHONO,Tomoaki, Tokyo 101-0021 (JP)
(74) Representative: MERH-IP Matias Erny Reichl Hoffmann
(86) International application number: PCT/JP2010/059503
(87) International publication number: WO 2010/143588

(57) **Abstract**

Disclosed is an ultrasonic diagnostic apparatus provided with an imaging unit configured to obtain an ultrasonic image of a carotid artery portion of an object to be examined, a parting line calculation unit configured to set at least one threshold value and calculate a parting line for segmenting the ultrasonic image into multiple regions, an intima-media thickness calculation unit configured to specify a direction for searching the multiple regions, search an intima-media region in the specified direction based on the brightness, draw multiple curves based on the position acquired by the search and positional information on the carotid artery portion in the ultrasonic image, and calculate the intima-media thickness from the distance between the multiple curves.

## Description

### Field of the Invention

The present invention relates to an ultrasonic diagnostic apparatus and the IMT (Intima-Media Thickness) measuring method therefore capable of measuring the IMT of an object to be examined.

### Description of Related Art

IMT measurement is known as being effective for detection of arterial sclerosis by acquiring an ultrasonic image of a carotid artery in an object to be examined and measuring the IMT on the acquired ultrasonic image. In IMT measurement, an operator sets a region of interest (ROI) on an ultrasonic image, extracts a lumen side boundary and an outer-membrane side boundary of an intima-media complex in a set ROI, and calculates the distance between the extracted boundaries.

An example of IMT measurement is disclosed in Non-Patent Document 1. In the method disclosed in Non-Patent Document 1, an operator first operates an ultrasonic diagnostic apparatus to obtain an ultrasonic image of a carotid artery portion in an object. Next, a processor cuts out a region including the carotid artery portion, and generates a blurred image. Then the processor executes the binarization process on the blurred image and extracts the carotid artery region. The processor further extracts an edge portion for extracting a contour from the carotid artery region. The processor then applies the snake method on the extracted edge portion, and extracts the contour of the intima-media and outer membranes in the carotid artery region by generating contour lines using the B-spline interpolation.

### Prior Art Documents

Non-Patent Document 1: C. P. Loizou et al. "Snakes based segmentation of the common carotid artery intima media", Med Bio Eng Comput (2007) 45:35-49

However, lowering of accuracy in boundary extraction at the time of IMT measurement attributed to interfusion of noise still remains as a problem even in Non-Patent Document 1.

For example, there are cases that noise content such as artifacts or speckle noise which is unique to ultrasonic waves generated from a lumen of a blood vessel or the outside of an outer membrane interfuses on the lumen-side boundary or the outer-membrane side boundary at the time of IMT measurement, thus IMT measurement with high accuracy cannot be achieved unless determination is made whether the pixel on the boundary is a signal or noise.

The objective of the present invention is to provide the ultrasonic diagnostic apparatus and the IMT measurement method capable of restraining influence of noise in boundary extraction at the time of IMT measurement.

### Brief Summary of the Invention

In order to achieve the above-described objective, the ultrasonic diagnostic apparatus of the present invention is characterized in comprising:
an imaging unit configured to obtain an ultrasonic image of a carotid artery portion in an object to be examined;
a parting-line calculation unit configured to calculate parting lines for segmenting the ultrasonic image into multiple regions by setting at least one threshold value; and
an intima-media thickness calculation unit configured to specify the direction for searching the multiple regions, search an intima-media region in the specified direction based on the brightness, draw multiple curves based on the information acquired by the search and positional information of the carotid artery portion in the ultrasonic image, and calculate the intima-media thickness based on the distance between the drawn curves.

Also, the IMT measurement method of the ultrasonic diagnostic apparatus related to the present invention is characterized in including:
a step of obtaining an ultrasonic image of a carotid artery portion in an object by an imaging unit;
a step of calculating parting lines for segmenting the ultrasonic image into multiple regions by setting at least one threshold value by a parting-line calculation unit; and
a step of specifying the direction for searching the multiple regions by an intima-media thickness calculation unit, searching an intima-media region in the specified direction based on the brightness, drawing multiple curves from the information acquired by the search and positional information of the carotid artery region in the ultrasonic image, and calculate the intima-media thickness based on the distance between the drawn curves.

As described above, in accordance with the ultrasonic diagnostic apparatus and the IMT measurement method of the present invention, it is possible to restrain the influence of noise in boundary extraction at the time of IMT measurement, since an ultrasonic image of a carotid artery portion of an object is obtained by an imaging unit, parting lines for segmenting the ultrasonic image into multiple regions are calculated by setting at least one threshold value by a parting line calculation unit, the direction for searching the multiple regions is specified, the intima-media region in the specified direction is searched based on the brightness, multiple curves are drawn from the information acquired by the search and positional information of the carotid artery portion in the ultrasonic image, and the intima-media thickness is calculated based on the distance between the drawn curves by an intima-media thickness calculation unit.

### Effect of the Invention

In accordance with the present invention, it is possible to provide the ultrasonic diagnostic apparatus and the IMT measurement method capable of restraining the influence of noise in boundary extraction at the time of IMT measurement.

### Brief Description of the Diagrams

Fig. 1 is a block diagram showing the general outline of the ultrasonic diagnostic apparatus in a first embodiment.
Fig. 2 is a display example of a monitor in IMT measurement by the ultrasonic diagnostic apparatus in the first embodiment.
Fig. 3 is a flowchart for explaining an example of operation procedure for IMT measurement by the ultrasonic diagnostic apparatus in the first embodiment.
Fig. 4A is a graph showing an example of brightness variation in the depth-direction of a certain row of pixels.
Fig. 4B is a graph showing an example of brightness variation in the depth-direction of a certain row of pixels.
Fig. 4C is a view showing an example of eliminating noise appearing on an image.
Fig. 4D is a view showing an example of eliminating noise appearing on an image.
Fig. 5A is an example of region segmentation.
Fig. 5B is a view showing an example of region segmentation.
Fig. 5C is a view showing an example of region segmentation.
Fig. 5D is a view showing an example of region segmentation.
Fig. 6A is a view showing an example of correction of a parting line on the lumen side and a parting line on the outer membrane side.
Fig. 6B is a view showing an example of correction of a parting line on the lumen side and a parting line on the outer membrane side.
Fig. 7A is a view showing the extraction method of boundary positions.
Fig. 7B is a view showing the extraction method of boundary positions.
Fig. 8A is a view showing the correction method of boundaries.
Fig. 8B is a view showing the correction method of boundaries.
Fig. 8C is a view showing the correction method of boundaries.
Fig. 9A is a view showing an example of region segmentation in a second embodiment.
Fig. 9B is a view showing an example of region segmentation in the second embodiment.
Fig. 9C is a view showing an example of region segmentation in the second embodiment.
Fig. 10A is a view showing an example of correction of a parting line in the second embodiment.
Fig. 10B is a view showing an example of correction of a parting line in the second embodiment.
Fig. 11A is a view showing the extraction method of a boundary position in the second embodiment.
Fig. 11B is a view showing the extraction method of a boundary position in the second embodiment.
Fig. 12 is a view showing boundary extraction in a third embodiment.
Fig. 13A is a view showing an example of a case that a rectangle-shaped ROI is tilted with respect to the incident direction of an ultrasonic wave.
Fig. 13B is a view showing an example of a case that an ROI is set in a parallelogram shape.
Fig. 13C is a view showing an example of a case that an ROI is set so that its both ends on the right and the left are parallel to the incident direction of an ultrasonic wave and its vertical ends are along the running direction of the carotid artery.
Fig. 14A is a view showing an example of a case that the extraction direction is set vertical to the running direction of a carotid artery.
Fig. 14B is a view showing an example of a case that an ROI is set by vertically matching its both ends on the right and the left with the running direction of a carotid artery and its vertical ends are along the running direction of the carotid artery.

### Detail Description of the Invention

Preferable embodiments of the ultrasonic diagnostic apparatus and the IMT measurement method related to the present invention will be described below referring to the attached diagrams. In the following description, the same function parts are represented by the same reference numerals, and the duplicative description thereof is omitted.

### Embodiment 1

In the first embodiment, region segmentation is performed by three values. Also, the first embodiment exemplifies the case that, in the ultrasonic image including a carotid region which is inputted at the timing synchronized with the biological signals, the lumen side boundary and the outer-membrane side boundary of an intima-media complex are extracted, and the IMT value is measured, displayed and outputted. Also in the present embodiment, the signals are of the brightness in the ultrasonic image, and the extraction position is the posterior wall of a carotid artery.

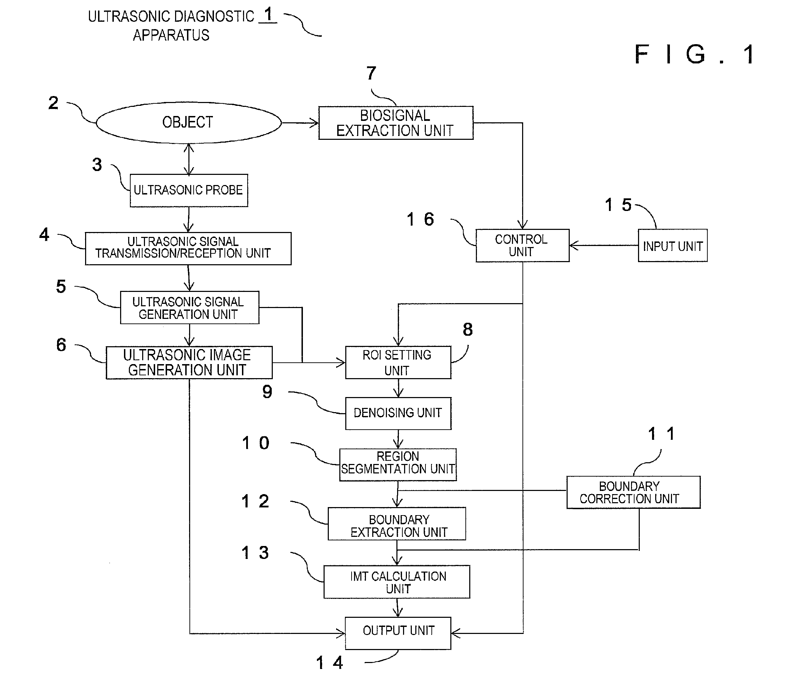

Fig. 1 is a block diagram showing the general outline of the ultrasonic diagnostic apparatus in the first embodiment. The ultrasonic diagnostic apparatus shown in Fig. 1 transmits ultrasonic waves to an object for making ultrasonic diagnosis using the reflected echo signals from the object.

The ultrasonic diagnostic apparatus comprises an ultrasonic probe 3, an ultrasonic transmission/reception unit 4, an ultrasonic signal generation unit 5, an ultrasonic image generation unit 6, a biosignal extraction unit 7, an ROI setting unit 8, a desnoising unit 9, a region segmentation unit 10, a boundary correction unit 11, a boundary extraction unit 12, an IMT calculation unit 13, an output unit 14, an input unit 15 and a control unit 16.

An ultrasonic probe 3 transmits ultrasonic waves to a target portion of an object 2 from transducers and receives the reflected echo signals from the object 2. There are three kinds of ultrasonic probe 3 that are a linear type, a convex type and a sector type depending on the intended use.

The ultrasonic transmission/reception unit 4 drives the ultrasonic probe 3 for executing transmission, switches the circuit from the state of transmission to reception for the ultrasonic probe 3 to execute reception, and transmits the reflected echo signals received by the ultrasonic probe 3 to the ultrasonic signal generation unit 5.

the ultrasonic signal generation unit 5 executes signal processing on the reception signal from the ultrasonic transmission/reception unit 4 according to the imaging setting set by the input unit 15 via a phasing circuit or an amplifying circuit so as to obtain a phased ultrasonic signal.

The ultrasonic image generation unit 6 generates an ultrasonic image from the signals inputted from the ultrasonic signal generation unit 5 based on the imaging setting set via the input unit 15.

The biosignal extraction unit 7 extracts biosignals of the object 2 and converts them into electrical signals. There are biosignals such as an ECG (electrocardiogram) or a PCG (phonocardiogram).

The ROI setting unit 8 sets an ROI on the signals in the ultrasonic image generated by the ultrasonic image generation unit 6. The ROI is manually set by an examinee using the input unit 15. Also, there are cases that the ROI is set in a computer program as disclosed, for example in JP-A-H11-155862. The case that the ROI is set in a computer is referred to as automatic setting of ROI.

The denoising unit 9 eliminates speckle noise, acoustic noise and heat noise that are overlapped on an image. Denoising improves extraction accuracy of the boundary extraction unit 12 by smoothing brightness variation of an image.

The region segmentation unit 10 calculates parting lines to be the boundary positions for the three regions of a lumen region, an intima-media region and an outer-membrane region or the two regions of a lumen side region and an outer-membrane side region, by the method for acquiring a threshold value based on the brightness distribution of an ROI.

The boundary correction unit 11 corrects the positions of boundaries in the boundary extraction unit 12 from the parting lines calculated by the region segmentation unit 10.

The boundary extraction unit 12 extracts a lumen side boundary and an outer-membrane side boundary based on the positions of the parting lines. For example, the boundary extraction unit 12 extracts the lumen side boundary in the range limited from the lumen side parting line to the lumen side as well as the outer-membrane side boundary in the range limited from the outer-membrane side parting line to the outer-membrane side.

The IMT calculation unit 13 calculates the distance between the lumen side boundary and the outer-membrane side boundary as the IMT value. The IMT calculation unit 13 further calculates the statistics such as the average value, the maximum value and the minimum value of the IMT values in the ROI.

The output unit 14 is a device such as a monitor which displays boundaries or measured values on a screen and a printer which outputs the displayed content on a measurement report. The monitor is an output device such as a CRT, liquid crystal monitor, plasma monitor or organic EL monitor for displaying information such as images, letters and graphics.

Input unit 15 is an interface for manual operation of measurement condition setting or ROI setting in IMT measurement by an ultrasonic diagnostic apparatus, and an input device such as a keyboard, trackball, switch and dial.

The control unit 16 controls the entire system and the execution timing of IMT measurement or the operation timing of the output unit 14 using the timing of the biosignals outputted from the biosignal extraction unit 7. For example, a CPU is used as the control unit 16.

Therefore, the ultrasonic probe 3, the ultrasonic transmission/reception unit 4, the ultrasonic signal generation unit 5 and the ultrasonic image generation unit 6 function as an imaging unit for obtaining an ultrasonic image of a carotid artery portion in an object.

Also, they function as a parting-line calculation unit for calculating parting lines for segmenting the ultrasonic image into multiple regions by setting at least one threshold value.

Also, the biosignal extraction unit 7, the ROI setting unit 8, the denoising unit 9, the region segmentation unit 10, the boundary correction unit 11, the boundary extraction unit 12, the IMT calculation unit 13, the output unit 14, the input unit 15 and the control unit 16 function as an intima-media thickness calculation unit. The intima-media thickness calculation unit specifies the direction for searching the multiple regions, searches an intima-media region in the specified direction based on the brightness, draws multiple curves from the information acquired by the search and positional information of the carotid artery portion in the ultrasonic image and calculates the intima-media thickness based on the distance between the multiple curves. In the present embodiment, two directions are specified for searching the multiple regions.

Next, an operation example of the present embodiment will be described referring to the diagrams.

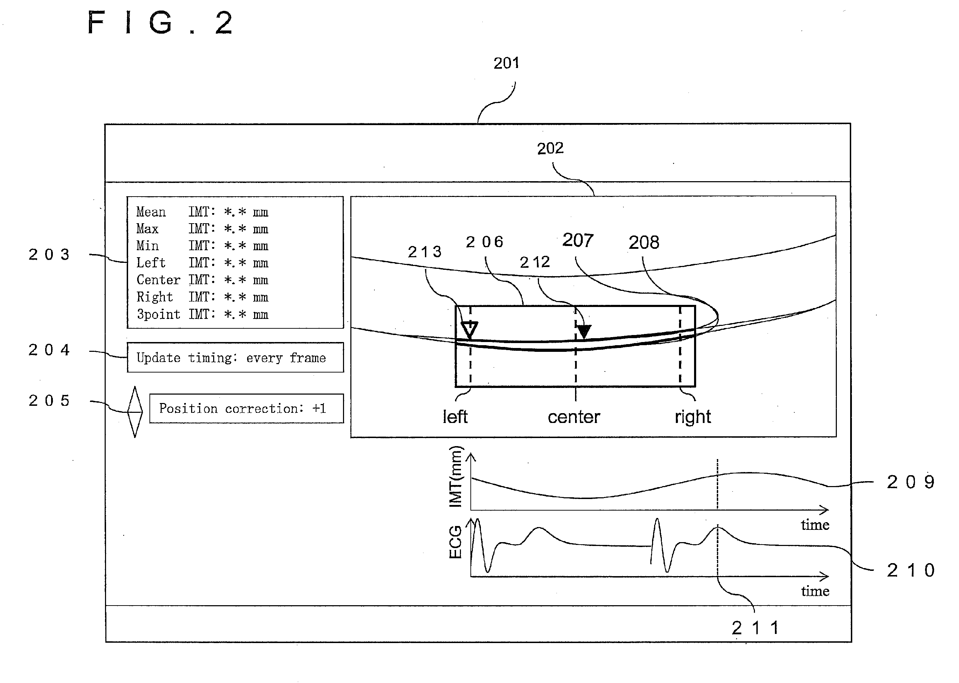

Fig. 2 is a display example of a monitor to be used for IMT measurement by the ultrasonic diagnostic apparatus in the first embodiment.

In Fig. 2, 201 is a screen, 202 is an image display region in the screen 201, 203 is a display region of IMT measurement values in the screen 201, 204 is an update timing display region in the screen 201, 205 is a display region of a parameter for positional correction in the screen 201, 206 is a region of interest (ROI) of the image display region 202, 207 is one end of a boundary in IMT measurement within the ROI 206, 208 is the other end of a boundary in IMT measurement within the ROI 206, 209 is a time curve of an IMT measurement value in a graph display region within the screen 201, 210 is an ECG curve in a graph display region within the screen 201, 211 is a line (displayed by a dotted line) showing the phase of a certain EGC (for example, an R-wave), 212 is a first measurement point in an IMT value and 213 is a second measurement point in an IMT value.

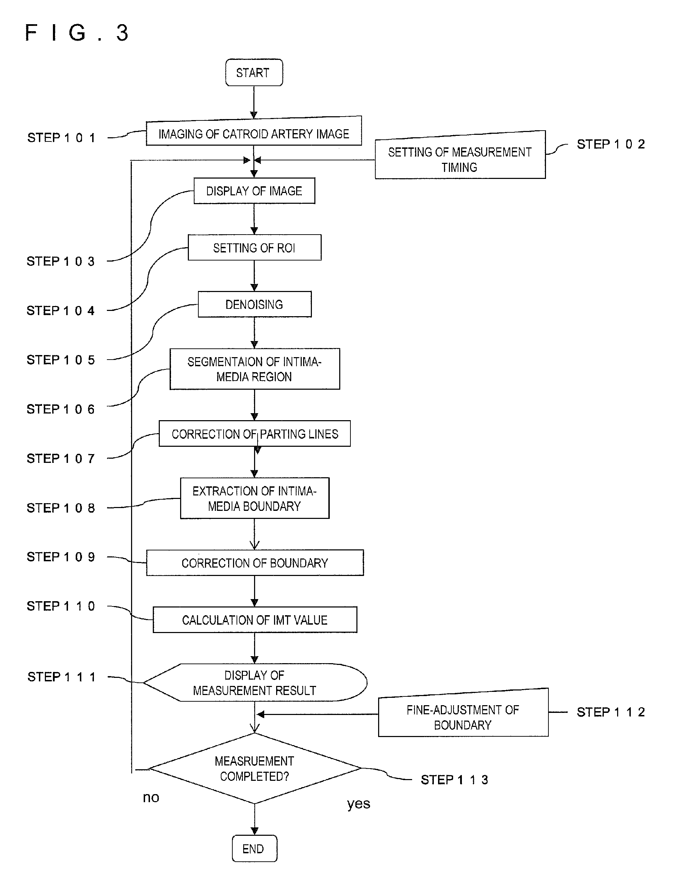

Fig. 3 is a flowchart showing an example of operation procedure of IMT measurement by the ultrasonic diagnostic apparatus in the first embodiment.
An operation example of IMT measurement by the ultrasonic diagnostic apparatus will be described according to the flow of processing shown in the flowchart of Fig. 3.

An examiner obtains an ultrasonic image including a carotid artery region by applying the ultrasonic probe 3 on a cervical region of the object 2. There are cases that the ultrasonic image including a carotid artery is referred to as merely an image (step 101: imaging of carotid artery image).

The examiner sets the measurement timing using the input unit 14. The setting example of measurement timing may be performed by setting an ECG as a biosignal and executing IMT measurement process for each R-wave of the ECG. Also, another setting example of measurement timing may be performed by setting time intervals of IMT measurement. In Fig. 2, an image is obtained, for example 30 frames per second, and the time interval is set for each frame. Setting of measurement timing is to be displayed on the update-timing setting condition display 204 on the display screen 201 in Fig. 2 (step 102: setting of measurement timing).

Next, the control unit 16 obtains an image at the set measurement timing from the images outputted from the ultrasonic image generation unit 6. The obtained image is displayed on the output unit 14 as image 202 in Fig. 2 (step 103: display of image).

Next, the examiner sets an ROI at the target position for IMT measurement on the carotid artery image 202 using the ROI setting unit 8. It is desirable to set an ROI at the position having less noise where the signal intensity of the lumen, the intima-media complex and the outer membrane is displayed continuously in the longitudinal direction of the blood vessel as well as stepwise in two steps in the short-axis direction thereof. Given this factor, the examiner sets an ROI using the input unit 15 while variably adjusting its size and position on the carotid artery image. While the shape of an ROI is set as a rectangle here, a circular shape or other shapes may be applied as long as it can be defined as a region. The automatic ROI setting method may also be used (step 104: setting of ROI).

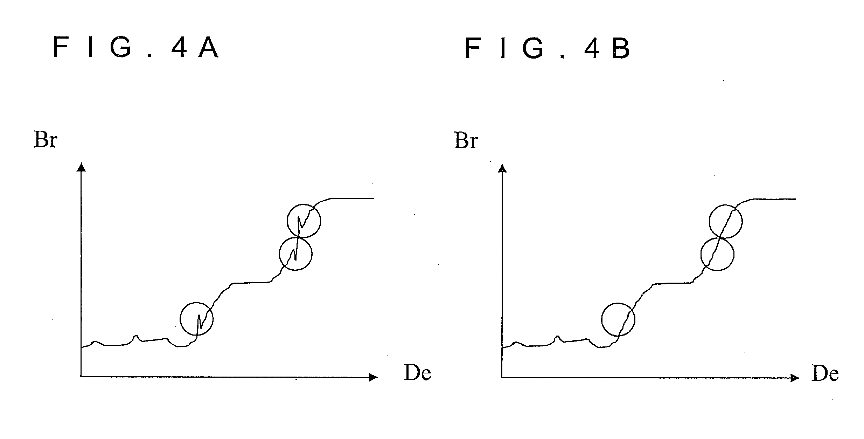

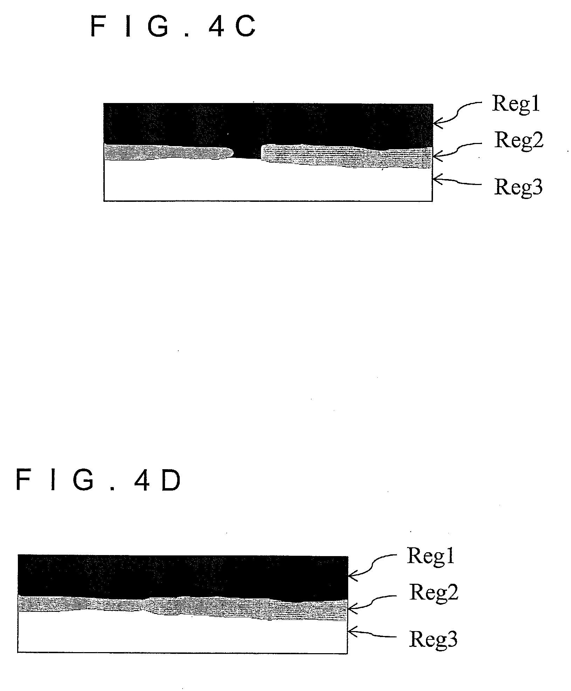

Next, the control unit 16 causes the denoising unit 9 to eliminate variation of the brightness value attributed to speckle noise, acoustic noise and heat noise overlapped on the image brightness value in the ROI. Concrete examples of denoising process will be described using Fig. 4A, Fig. 4B, Fig. 4C and Fig. 4D. Fig. 4A and Fig. 4B show an example of a process for eliminating noise that appears on a profile. Fig. 4A and Fig. 4B are graph examples showing variation of a certain pixel row in an ROI in the depth direction (De) of a brightness value (Br). Fig. 4A exemplifies the case that the brightness has local maximum values attributed to spike-like noise and those values are erroneously extracted as boundaries, as shown in circular marks on the graph. Given this factor, the median filtering is applied for denoising such local maximum values as shown in Fig. 4A. As the result of denoising, Br in the ROI with respect to De has smooth brightness variation as shown in the circular marks of Fig. 4B.

Fig. 4C and Fig. 4D are examples of eliminating noise that appears on an image. Fig. 4C shows the case that region segmentation is executed by segmenting into three regions of lumen region Reg1, intima-media region Reg2 and outer-membrane region Reg3, and intima-media region Reg2 cannot be segmented as one region because it is discontinued attributed to generation of acoustic noise. Discontinuity of a region attributed to noise as shown in Fig. 4C is often caused by acoustic noise. For eliminating acoustic noise, closing calculation of, for example the moving-average filter or morphology is used. The moving-average filter is the calculation performed by acquiring and smoothing time-series image data. Closing calculation of morphology is the calculation that interpolates structural elements exists in an object in an image so as to fill in small holes or cracks that are smaller than the structural elements. As a result of eliminating acoustic noise, the region in which the discontinuity in the lateral direction is filled as shown in Fig. 4D can be acquired.

Also, fine noises attributed to heat can be eliminated by applying a smoothing filter such as a moving-average filter

### (step 105: denoising)

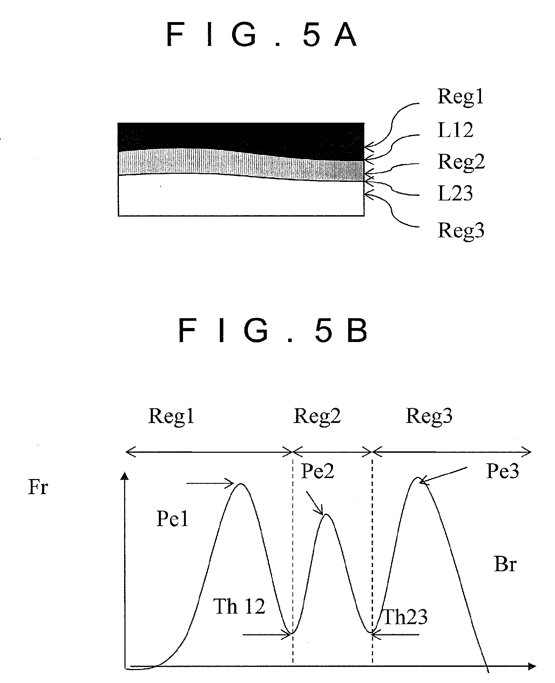

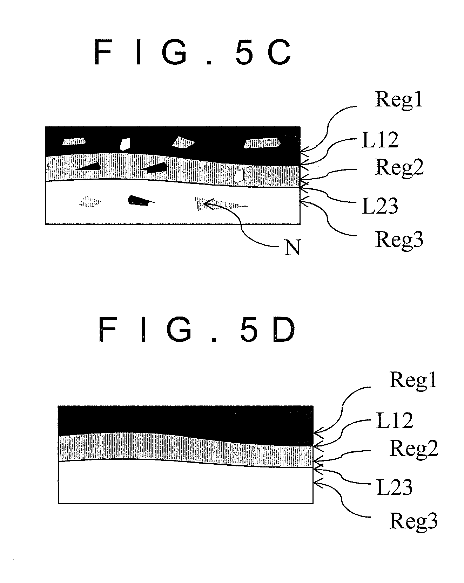

Next, the control unit 16 segments the intima-media region using the image brightness value within the ROI by the region segmentation unit 10, and extracts the parting lines. Extraction of the parting lines will be described using Fig. 5A, Fig. 5B, Fig. 5C and Fig. 5D. Fig. 5A, Fig. 5B, Fig. 5C and Fig. 5D are views showing and example of region segmentation. Fig. 5A is a view showing an example of regions and parting lines. Generally, lumen region Reg1 has the lowest brightness value in a carotid artery image, then intima-media region Reg2 and outer-membrane region Reg3 has the highest brightness value.

Given this factor, the region segmentation method for segmenting into three regions of lumen side region Reg1, intima-media region Reg2 and outer-membrane region Reg3 will be described. In Fig. 5B, frequency Fr of brightness value Br in a rectangle shaped ROI of Fig. 5A is calculated as a histogram and graphically displayed. The histogram has three independent regions, thus has local maximum values Pe1, Pe2 and Pe3 respectively for each region. Therefore, a first threshold value Th12 should be provided at the position of the local minimum value between Pe1 and Pe2, and a second threshold value Th23 should be provided at the position of the local minimum value between Pe2 and Pe3. Lumen region Reg1 and outer-membrane region Reg3 are segmented by the first threshold value Th12 and the second threshold value Th23, and the position where the brightness value from lumen region Reg1 to intima-media region Reg2 changes becomes a lumen side parting line L12.

Also, the position where the brightness value from intima-media region Reg2 to outer-membrane region Reg3 changes is set as an outer-membrane side parting line 23. As for the threshold value calculation, the method such as calculating the minimum value, the percentile method and the discriminant analysis method can be applied. The percentile method sets the threshold value so that the pixel number ratio between the 0-part and a part of a binarized or multi-valued image becomes p:1-p. Also, the discriminant analysis method determines the threshold so that the between-class variation of each region becomes the maximum when an image is binalized or muti-valued. Fig. 5C shows the result of region segmentation, wherein a region is segmented into lumen region Reg 1, intima-media region Reg2 and outer-membrane region Reg3 by the first threshold Th 12 value and the second threshold value Th 23. However, there are cases that specks of noise N remain attributed to heat noise as shown in Fig. 5C. The area of noise N is smaller compared to lumen region Reg1, intima-media region Reg2 and outer-membrane region Reg3.

Given this factor, the control unit 16 calculates magnitude correlation between the area of lumen region Reg1, intima-media region Reg2 and outer-membrane region Reg3 and the area of noise N. Further, the control unit 16 eliminates noise N while remaining lumen region Reg1, intima-media region Reg2 and outer-membrane region Reg3 having large areas. In this manner, noise N is eliminated as shown in Fig. 5D. Then the control unit 16 respectively calculates lumen side parting line L12 and outer-membrane side parting line L23 from the lumen region Reg1, intima-media region Reg2 and outer-membrane region Reg3 from which noise N is eliminated. Lumen side parting line L12 and outer-membrane side parting line L23 are respectively set as a lumen-side parting line and an outer-membrane side parting line (step 106: segmentation of intima-media region).

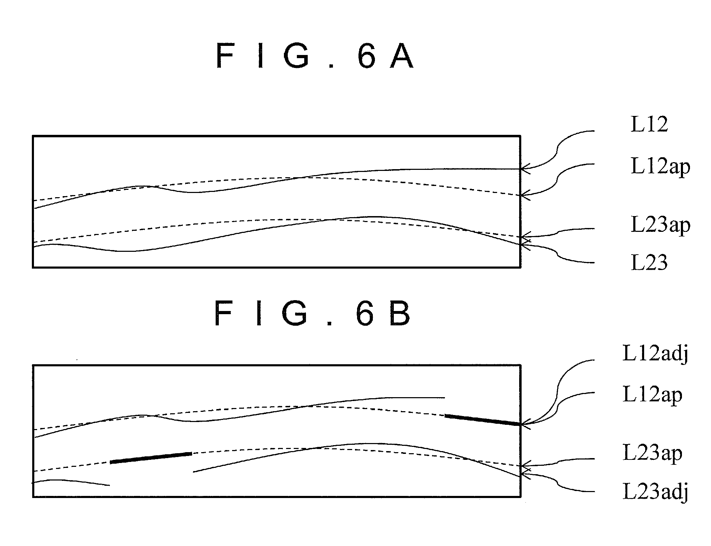

Next, the control unit 16 causes the boundary correction unit 11 to correct the positions of parting lines as shown in the examples of Fig. 6A, Fig. 6B and Fig. 6c. Fig. 6A, Fig. 6B and Fig. 6C are views showing examples of correction of lumen side parting line L12 and outer-membrane side parting line 23. Lumen side parting line 12 and outer-membrane side parting line 23 calculated in step 106 are drawn by solid lines in Fig. 6A. There are cases that lumen side parting line L12 and outer-membrane side parting line L23 are not coincided with the appropriate parting positions, since they still have precipitousness attributed to the noise which causes positional errors. Thus the precipitousness needs to be corrected. For the correction of lumen side parting line L12 and outer-membrane side parting line 23, the coordinate values on an image are calculated by the boundary correction unit 11, and approximated curve 12ap of the lumen side parting line and approximated curve 23ap of the outer-membrane side parting line that are shown by dotted lines in Fig. 6A are respectively created using the calculated coordinate values. As for creation of approximated curve L12ap of the lumen side parting line and approximated curve L23ap of the outer-membrane parting line, for example an approximation method such as the moving-average filter or polynomial approximation can be used. Then the control unit 16 calculates the distance between lumen side parting line L12 and approximated curve L12ap of the lumen side parting line.

Further, the control unit 16, in the case that the calculated distance is larger than the reference value, replaces the position of lumen side parting line 12 with the position of approximated curve L12ap of the lumen side parting line and sets it as corrected curve L12adj of the lumen side parting line. The reference value is inputted by an examiner via the input unit 15, and stored by the control unit 16 in a device such as a memory which is not shown in the diagram to be used for control operation. The part where lumen side parting line L12 and approximated line L12ap of the lumen side parting line are separated as shown in Fig. 6B is replaced with the position of a thick-line part on approximated curve L12ap of the lumen side parting line and set as corrected curve L12adj of the lumen side parting line. In this manner, the position of lumen side parting line L12 can be corrected by deletion of the extreme precipitousness. In the same way, the position of outer-membrane side parting line 23 can also be corrected using approximated curve L23ap of the outer-membrane side parting line and setting corrected curve L23ap of the outer-membrane side parting line (step 107: correction of parting lines).

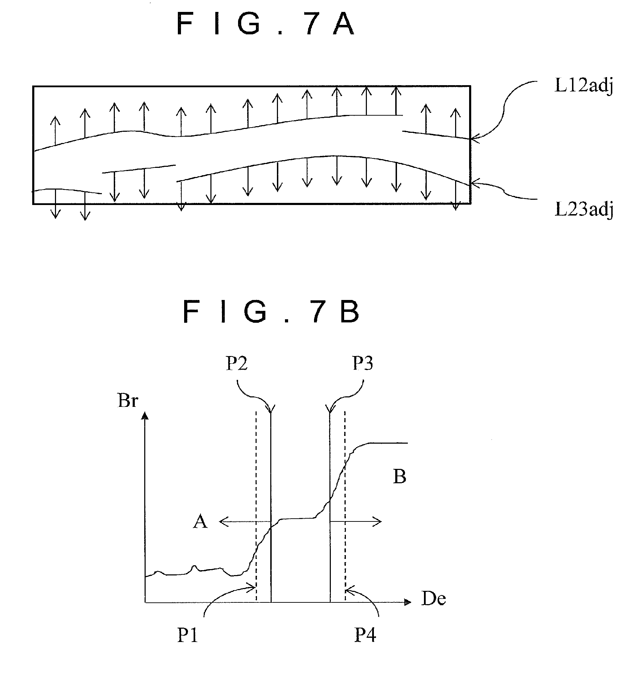

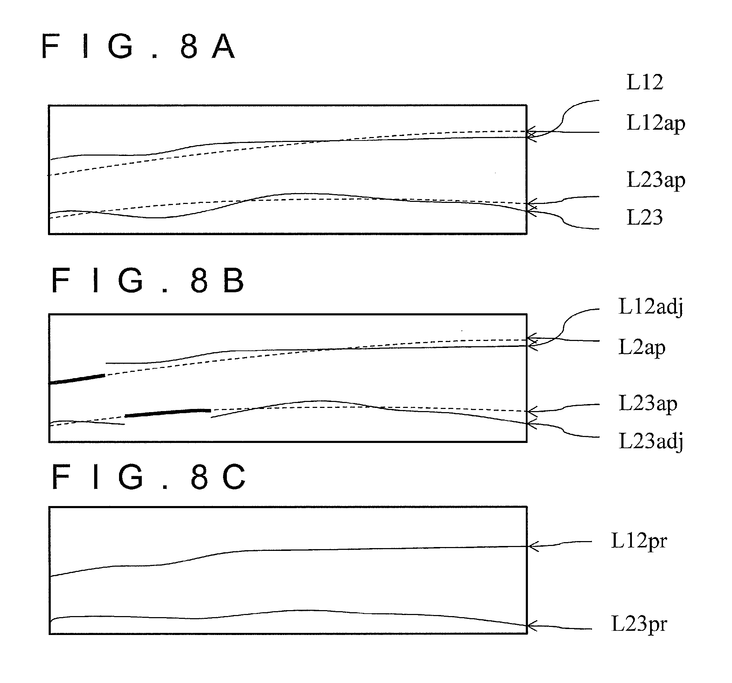

Next, the controller 16 causes the boundary extraction unit 12 to extract the lumen side boundary and the outer side boundary of inner-media region Reg2. Fig. 7 and Fig. 8 are views for explaining the extraction method of boundary positions. The solid lines in Fig. 7A are corrected curve L12adj of the lumen side parting line and corrected curve L23adj of the outer-membrane side parting line extracted in step 107. On the basis of the positions of corrected curve L12adj of the lumen side parting line and corrected curve L23adj of the outer-membrane parting line, boundary extraction for the lumen side boundary is performed by limiting the range from the lumen side parting line toward the lumen side as indicated by the arrows. Also the outer-membrane side boundary is extracted by limiting the range from the outer-membrane side parting line toward the outer-membrane side as indicated by the arrows. Fig. 7B shows the brightness variation in a cross-section of a certain row in Fig. 7A. The brightness is varied stepwise, and lumen side parting position P2 and outer-membrane side parting position P3 are respectively set at the positions where the brightness drastically changes on both sides of step-wise change (solid lines). The boundary extraction is executed respectively on the lumen side and the outer-membrane side from the positions of lumen side parting position P2 and outer-membrane side parting position P3, and lumen side boundary position P1 and outer-membrane side boundary position P4 denoted by dotted lines are extracted. As for the boundary extraction method, the method for extracting the position where the brightness variation reaches the maximum such as the derivative can be used. The boundary extraction method is applied to all rows of an ROI for extracting the boundary position. Assembly of these boundary positions is set as the lumen side boundary and the outer-membrane side boundary (step 108: extraction of intima-media boundary).

Next, the controller 16 causes the boundary correction unit 11 to correct the positions of the lumen side boundary and the outer-membrane side boundary. The correction method is the same as the correction method of parting lines in step 107. Fig. 8A, Fig. 8B and Fig. 8C are views for explaining the correction method of boundaries. The solid lines in Fig. 8A indicate the boundaries extracted in step 108. These boundaries often have precipitousness in the case that they are erroneously extracted at the positions of noise. Thus the precipitousness needs to be eliminated.

Fig. 8A and Fig. 8B are views showing an example that the processing of parting lines in Fig. 6A and Fig. 6B is applied to boundaries. A smoothing process such as the moving-average filter is executed so as to eliminate discontinuities such as the boundary parts between the thick line and the solid line in Fig. 8B and to acquire smooth boundaries L12pr and L23pr as shown in Fig. 8C (step 109: correction of boundaries).

Next, the control unit 16 causes the IMT calculation unit 13 to calculate the IMT value based on the distance between boundaries L12pr and L23pr. The IMT value is calculated as the distance between the lumen side boundary and the outer-membrane side boundary. As for the calculation values, the average value of an entire IMT boundary, the maximum value, the minimum value, the values of the left end, the center and the right end of the ROI or the average value of the left end, the center and the right end of the ROI are acquired (step 110: calculation of IMT value).

Next, the control unit 16 causes the output unit 14 to display the boundaries or the IMT measurement values on the display screen 201. Lumen side boundary 207 and outer-membrane side boundary 208 are displayed by being superimposed on the ROI 206 in the carotid artery image 202. The dotted lines indicating the left end, the center and the right end of the ROI, are displayed in ROI 206 so that the examinee can move them to the left and the right using an input device. The maximum value and the minimum value of the IMT value are displayed by marking the positions at the values thereof. For example, the marks are displayed at position 212 where the maximum value is and at position 213 where the minimum value is while being superimposed on the ROI 206. The measurement values are displayed by tabular form 203. The IMT value is displayed on graph 209, since it varies over time. Further, since an IMT value is to be synchronized with a biosignal, graph 210 of a biosignal is displayed parallel to the graph of the IMT, and bar 211 is displayed at the phase which is presently measured (step 111: display of measurement result).

Next, the examinee performs fine adjustment of boundary positions using the input unit 15. There are cases that the process for correcting noise is not sufficient enough for correction of the extracted boundaries. At this time, the examiner manually or semi-automatically performs fine adjustment on the boundary positions while observing the boundaries displayed in step 111. The manual adjustment is performed using the input unit 15. For example, fine adjustment is performed by dragging and dropping a part of the boundary. The semi-automatic adjustment is performed by changing the processing parameter related to the boundary such as the coordinates of the boundary, the threshold value for region segmentation, the threshold value for boundary correction and the boundary extraction range (step 112: fine adjustment of boundary).

Next, the control unit 16 determines whether the measurement is completed or not (step 113). In the case that the measurement is to be continued, after waiting for the measurement timing set in step 102, step 103 ∼ step 112 are repeatedly executed (step 113: determination of measurement completion).

In accordance with the present embodiment, it is possible to restrain the influence of noise in boundary extraction for IMT measurement.

Also, the characteristic effect in the present embodiment is that the boundaries which are interconnected in the longitudinal direction of a blood vessel can be extracted without discontinuity since parting lines are initially extracted. Further, the boundary extracting range can be limited on the basis of parting positions, thus restriction of noise can be performed without being influenced by the noise which is far from the boundary. Even when influenced by noise, correction can be made by the boundary correction process. Also, by semi-automatic boundary adjustment which changes the boundary processing parameter, the examiner can adjust the boundary as desired.

### Embodiment 2

In the second embodiment, region segmentation is executed by binarization, not by three-valued processing. The apparatus configuration in Fig. 1 and the flowchart described in Fig. 3 except step 106 ∼ step 108 are the same as in the first embodiment. Only step 106 ∼ step 108 will be described in the second embodiment.

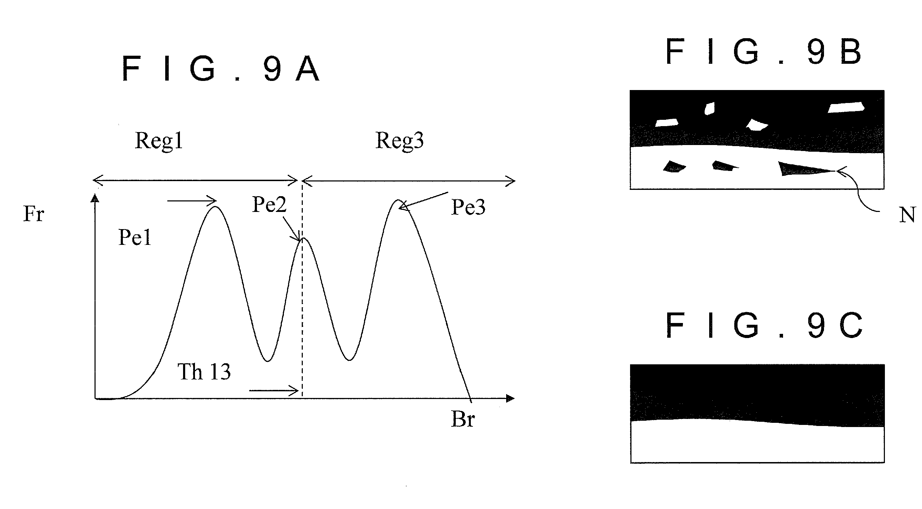

The control unit 16 causes the region segmentation unit 10 to segment an ROI into two regions of a lumen side region and an outer-membrane side region using the image brightness value within the ROI. Extraction of parting lines will be described using Fig. 9A, Fig. 9B and Fig. 9C. Fig. 9A, Fig. 9B and Fig. 9C are views showing an example of region segmentation in the second embodiment. Fig. 9A is a view for explaining regions and a parting line. In Fig. 9B, frequency Fr of brightness value Br in a rectangle shaped ROI of Fig. 5A is calculated as a histogram and graphically displayed. Generally, lumen region Reg1 has the lowest brightness value in a carotid artery image, and outer-membrane region Reg3 has the higher brightness value. Given this factor, the method for segmenting into two regions of lumen region Reg1 and outer-membrane region Reg3 will be described. The histogram has three independent regions, thus has local maximum values Pe1, Pe2 and Pe3 respectively for each region. Lumen side region Reg1 includes a lumen and a part of the intima-media region.

Also, outer-membrane side region Reg3 includes a part of the intima-media region and the outer-membrane region. Since the two regions have different brightness respectively, the region is segmented into two by providing threshold value Th13 to the brightness value. Threshold value Th13 should be provided at the position of the apex of local maximum value Pe2. The region can be segmented into two regions by executing binarization using threshold value Th13. As for the threshold determination method, the method such as extracting the local maximum value of the center part in the diagram, the percentile method or the discriminant analysis method can be applied. The result of region segmentation shown in Fig. 9B can be acquired by binarizing with threshold value Th13. However, there are cases that segmentation is executed in fine noise regions influenced by noise. Since these noise regions are smaller than lumen side region Reg1 and outer-membrane side region Reg3, the smaller regions are to be deleted by remaining lumen side region Reg1 and outer-membrane side region Reg3 having large areas. In this manner, the ROI is segmented into lumen side region Reg1 and outer-membrane side region Reg3 as shown in Fig. 9C. Then the parting line for lumen side region Reg1 and outer-membrane side region Reg3 is extracted (step 106: segmentation of intima-media region).

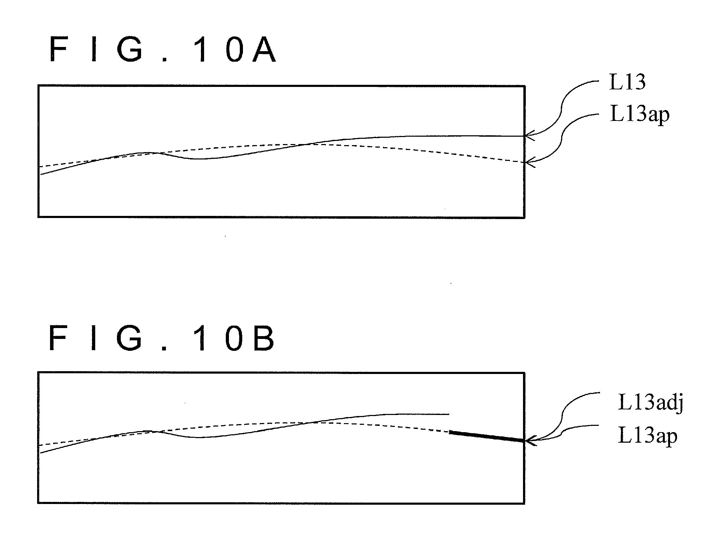

The control unit 16 causes the boundary correction unit 11 to correct the position of the parting line as shown in an example of Fig. 10A and Fig. 10B. Fig. 10A and Fig. 10B are views showing an example of correcting parting line L13 in the second embodiment. Parting line L13 calculated in step 106 is denoted by a solid line in Fig. 10A. There are cases that lumen side parting line L13 is not coincided with the parting position, since it still has precipitousness attributed to noise which causes positional error. For correction of parting line L13, the boundary correction unit 11 calculates the coordinate value on the image, and creates approximated curve L13ap of the parting line denoted by a dotted line in Fig. 10A using the calculated coordinate value. For creation of approximated curve L13ap of the parting line, the approximation method such as a moving average filter or polynomial approximation can be used. Then the distance between the parting line L13 and approximated curve L13ap of the lumen side parting line is calculated, the position of parting line L13 is replaced with the position of approximated curve L13ap of the parting line when the calculated distance is larger than the reference value, and the replaced curve is set as corrected curve L13adj of the parting line. The reference value is stored by the examinee using the input unit 15 in a device such as a memory which is not shown in the diagram, to be used by the control unit 16. As shown in Fig. 10B, the part where parting line L13 and approximated curve L13ap of the parting line are apart is replaced with the position of a thick part on approximated curve L13ap of the parting line, and set as corrected curve 13adj of the parting line. In this manner, extreme precipitousness of parting line L13 is eliminated and the position of parting line L13 can be properly corrected (step 107: correction of parting lines).

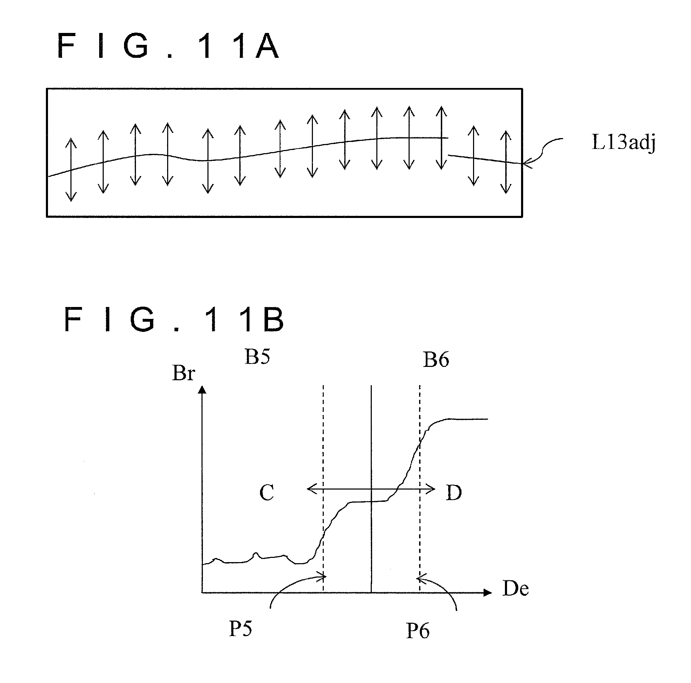

Next, the control unit 16 causes the boundary extraction unit 12 to extract lumen side boundary B5 and outer-membrane side boundary B6 of an intima-media region. Fig. 11A and Fig. 11B are views for explaining the extraction method of boundary positions in the second embodiment. The solid line in Fig. 11A is parting line L13adj which is extracted in step 107. Boundary extraction of lumen side boundary B5 is executed on the basis of the position of parting line L13adj by limiting the range from the parting line toward the lumen side as shown by an arrow A. Also, outer-membrane side boundary B6 is extracted by limiting the range from the parting line toward the outer-membrane side as shown by an arrow B.

Fig. 11B shows the brightness variation in a cross-section of a certain row. The brightness is varied stepwise in two steps, and the parting position is determined at the center of the steps. From that position, boundary extraction is executed toward the lumen side and the outer-membrane side respectively so as to extract lumen side boundary position P5 and outer-membrane side position P6 shown by dotted lines (step 108: extraction of intima-media boundary).

In accordance with the present embodiment, it is possible to restrain the influence of noise in boundary extraction for IMT measurement.

The characteristic effect of the present embodiment is that calculation amount for region segmentation and correction of parting lines can be reduced since only one threshold value needs to be calculated while two threshold values are necessary in the first embodiment.

### Embodiment 3

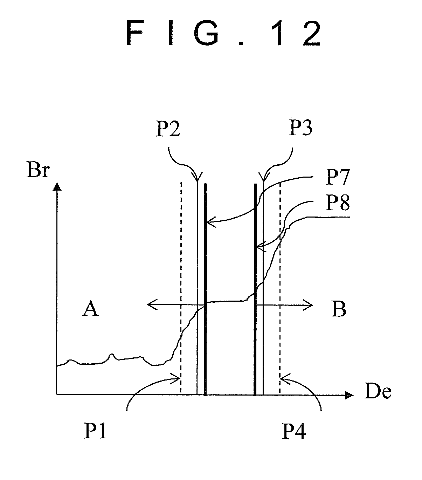

The third embodiment enables re-setting of a threshold value by freezing an image (a 3-valued image in the first embodiment, and a binarized image in the second embodiment). Also, the third embodiment is another modified example of step 108. Fig. 12 is a view for explaining boundary extraction in the third embodiment.

The thick lines in Fig. 12 show the positions for newly starting boundary extraction that are changed from P2 of the first embodiment to P7 and from P3 of the first embodiment to P8 respectively. The amount for changing the positions are manually adjusted each time using boundary fine-adjustment button 205. For example, if the amount of change is set in the negative direction the starting position is set toward a media region as shown in Fig. 12, and if the amount of change is set in the positive direction the starting position is set toward a lumen side for an outer-membrane side boundary and toward an outer-membrane side for a lumen side boundary. Fig. 12 is a view showing the case that boundary extraction is executed from the halfway of media region toward the lumen or the outer-membrane by changing the boundary extraction starting position in the negative direction. Fig. 12 shows a case that a boundary extracting position is set in the negative direction, and boundary extraction is executed from the midway of a media region toward a lumen or an outer membrane. Also, the starting points may be set for the outer-membrane side boundary and the lumen side boundary separately. The setting range of the amount of change is within the range where the starting positions of the outer-membrane side and of the lumen side are not overlapped.

In accordance with the present embodiment, it is possible to restrain the influence of noise in boundary extraction for IMT measurement.

Also, characteristic effect of the present embodiment is that boundary extraction at a position with higher accuracy can be executed, since boundary extraction stating positions can be set while avoiding boundary positions, thereby preventing deviance of extraction.

### Embodiment 4

In the first ∼ third embodiments, the direction for boundary extraction is set parallel to the right and the left sides (narrow sides) of a rectangle which is set as an ROI. The method in the present embodiment sets the direction for boundary extraction in accordance with the running direction of a carotid artery or the direction of a probe. Step 104 and step 108 of which the processing content is different from the first embodiment will be described.

The examiner sets an ROI at a target position for IMT measurement on carotid artery image 202 using the ROI setting unit 8. The difference from the first embodiment is to modify the shape of an ROI. The examiner adjusts the shape of an ROI using the input unit 15. Then the examiner sets the extracting direction in accordance with the shape of ROI or the running direction of a carotid artery in step 108.

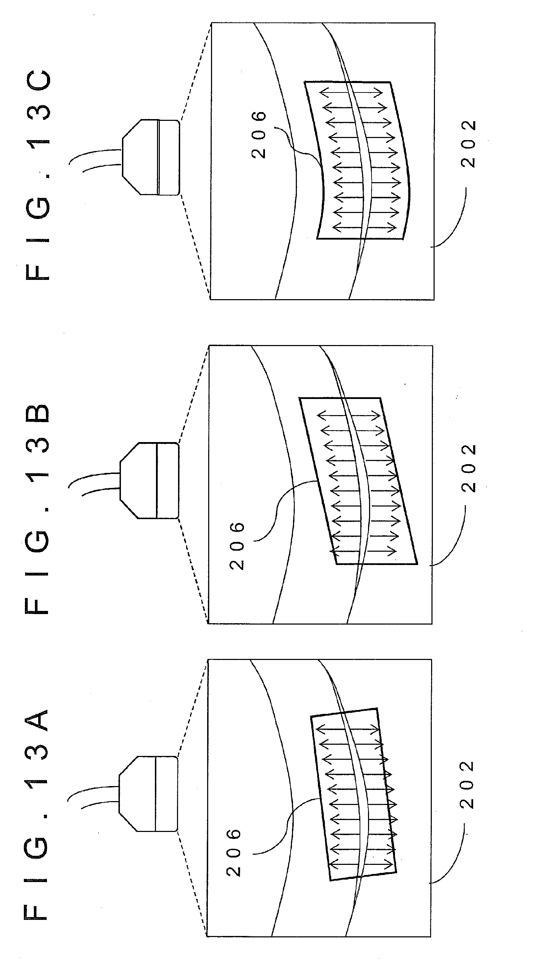

Fig. 13A is a view showing an example of a case that a rectangle-shaped ROI is tilted with respect to the incident direction of an ultrasonic wave, whereby the extracting direction is parallel to the incident direction. The extracting direction is set parallel to the incident direction regardless of the direction of the sides of a rectangle. In this case, extraction process can be executed simply and quickly since the brightness aligned in a reticular pattern is to be acquired as it is in the longitudinal direction.

Fig. 13B is view showing an example of a case that an ROI is set in the shape of a parallelogram, wherein the right and the left sides of an ROI are set to be parallel to the incident direction of an ultrasonic wave. In this case, since the right and the left sides of an ROI are set parallel to the incident direction of an ultrasonic wave, it is possible to set a sufficient length of extraction range over the entire ROI.

Fig. 13C is a view showing an example that the right and the left sides of an ROI are set parallel to the incident direction of an ROI and the top and the bottom sides of the ROI are set along the running direction of a carotid artery. In this case, since the top and the bottom sides of an ROI are along the running direction of a carotid artery, it is possible to set a sufficient length of extraction range over the entire ROI.

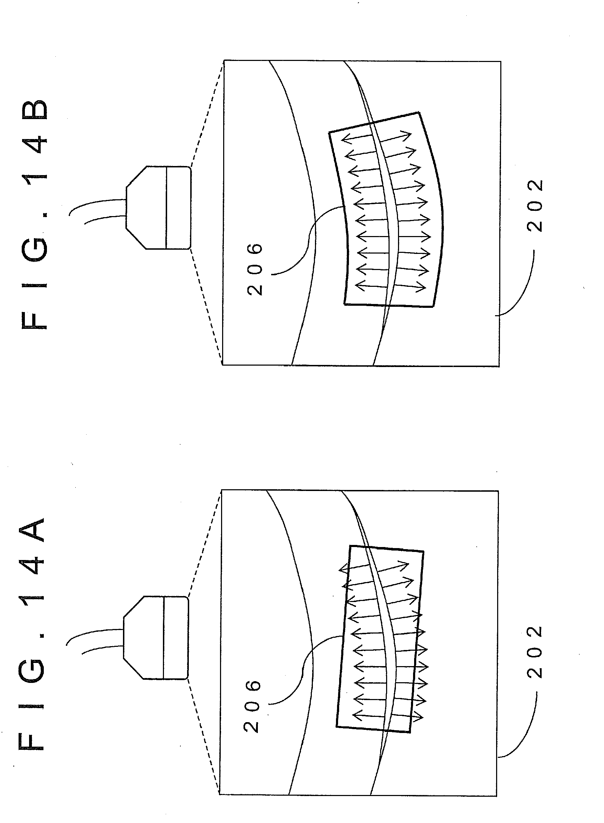

Fig. 14A is a view showing an example of a case that the extracting direction is set vertical to the running direction of a carotid artery. In other words, the extracting direction is set vertical to the lumen side or the outer-membrane side shown in Fig. 14A. In this case, the direction for extracting a boundary and the blood vessel wall are vertical to each other, thus higher accuracy in calculation of boundary extraction can be achieved compared to the boundary extraction in the oblique direction in the other embodiments.

Fig. 14B is a view showing an example of a case that the right and the left sides of an ROI are set vertical to the running direction of a carotid artery and the top and the bottom sides of the ROI are set along the running direction of the carotid artery. In this case, since the right and the left sides of an ROI are parallel to the extracting direction and the top and the bottom sides of the ROI are vertical to the running direction of the carotid artery, it is possible to set a sufficient length of measurement range in the entire ROI and highly accurate calculation for boundary extraction can be executed.

The present invention can be applied to an ultrasonic wave signal outputted from the ultrasonic signal generation unit 5, and also to a carotid artery anterior wall which is diphycercal to the carotid artery exterior wall simply by reversing the extracting direction.

In accordance with the present embodiment, it is possible to restrain the influence of noise in boundary extraction for IMT measurement.

Also, characteristic effect of the present embodiment is that boundary extraction at more accurate position can be executed since an ROI can be set as more fitting and sufficient shape in accordance with the running direction of a carotid artery or the direction of a probe.

The preferable embodiments of the ultrasonic diagnostic apparatus, etc. according to the present invention have been described. However, the present invention is not limited to these embodiments. It is obvious that persons skilled in the art can make various kinds of alterations or modifications within the scope of the technical idea disclosed in this application, and it is understandable that they belong to the technical scope of the present invention.

### Description of Reference Numerals

1: ultrasonic diagnostic apparatus, 2: object, 3: ultrasonic probe, 4: ultrasonic signal transmission/reception unit, 5: ultrasonic signal generation unit, 6: ultrasonic image generation unit, 7: biosignal extraction unit, 8: ROI setting unit, 9: denoising unit, 10: region segmentation unit, 11: boundary correction unit, 12: boundary extraction unit, 13: IMT calculation unit, 14: output unit, 15: input unit, 16: control unit

## Claims

1. An ultrasonic diagnostic apparatus comprising:
an imaging unit configured to obtain an ultrasonic image of a carotid artery portion in an object to be examined:
a parting line calculation unit configured to set at least one threshold value and calculate a parting line for segmenting the ultrasonic image into multiple regions;
an intima-media thickness calculation unit configured to specify the direction for searching the multiple regions, search an intima-media region in the specified direction based on the brightness, draw multiple curves from the position acquired by the search and positional information of the carotid artery portion in the ultrasonic image and calculate the intima-media thickness based on the distance between the multiple curves.

2. The ultrasonic diagnostic apparatus according to claim 1, **characterized in** further comprising a biosignal measurement unit configured to measure a biosignal of the object, wherein the intima-media thickness measurement unit calculates the intima-media thickness using the measured biosignal and the distance.

3. The ultrasonic diagnostic apparatus according to claim 1, wherein the parting line calculation unit segments the ultrasonic image into a blood vessel lumen region, an intima-media complex region and an outer-membrane region using a first threshold value and a second threshold value.

4. The ultrasonic diagnostic apparatus according to claim 3, wherein the boundary extraction unit extracts a lumen side boundary from a lumen side parting line in the lumen side direction and an outer-membrane side boundary from the outer-membrane side parting line in the outer-membrane side direction.

5. The ultrasonic diagnostic apparatus according to claim 1, wherein the parting line calculation unit segments the ultrasonic image into a blood vessel lumen side region and an outer-membrane side region using a first threshold value.

6. The ultrasonic diagnostic apparatus according to claim 1, wherein the parting line calculation unit calculates a parting line based on the area in the respective regions.

7. The ultrasonic diagnostic apparatus according to claim 6, wherein the parting line calculation unit calculates a parting line so that the area of each region reaches the maximum.

8. The ultrasonic diagnostic apparatus according to claim 6, wherein the parting line calculation unit deletes the region of which the area is not the maximum.

9. The ultrasonic diagnostic apparatus according to claim 1, wherein the boundary extraction unit sets the direction for extracting a lumen side boundary and an outer-membrane side boundary in the vertical direction to the irradiation of an ultrasonic wave and/or the running direction of a carotid artery.

10. The ultrasonic diagnostic apparatus according to claim 1, **characterized in** further comprising a position correction unit configured to calculate the positional coordinates of the parting line and/or a boundary, generate an approximated curve from the positional coordinates and correct the position of the parting line and/or the boundary from the approximated curve.

11. The ultrasonic diagnostic apparatus according to claim 4, wherein the boundary extraction unit extracts the position where the brightness variation in each region from the lumen side toward the outer-membrane side reaches the positive maximum as a boundary.

12. The ultrasonic diagnostic apparatus according to claim 4, wherein the boundary extraction unit extracts the position where the brightness variation in each region reaches the positive maximum along the irradiating direction of an ultrasonic wave and/or the direction vertical to the running direction of a carotid artery as a boundary.

13. The ultrasonic diagnostic apparatus according to claim 10, wherein the position correction unit further comprises a user interface unit capable of adjusting the position of the boundary while the ultrasonic image is in a frozen state.

14. An intima-media thickness measurement method of an ultrasonic diagnostic apparatus including:
a step of obtaining an ultrasonic image of a carotid artery in an object to be examined by an imaging unit;
a step of calculating a parting line for segmenting the ultrasonic image into multiple regions by setting at least one threshold by a parting line calculation unit;
a step of specifying the direction for searching the multiple regions, searching an intima-media region in the specified direction based on the brightness, drawing multiple curves from the position acquired by the search and positional information of the carotid artery in the ultrasonic image and calculating the intima-media thickness based on the distance between the multiple curves by the intima-media thickness calculation unit.
